(19) Europäisches Patentamt
European Patent Office
Office européen des brevets

(11) EP 1 029 864 B1

(12) EUROPEAN PATENT SPECIFICATION

(45) Date of publication and mention of the grant of the patent: **24.09.2003 Bulletin 2003/39**

(51) Int Cl.[7]: **C07D 501/18**, C07D 501/12

(21) Application number: **00111498.2**

(22) Date of filing: **21.12.1992**

# (54) Process for the purification of a 3-cephem-4-carboxylic acid derivative

Verfahren zur Reinigung von einem 3-Cephem-4- Carbonsäurederivat

Procédé de purification d'un dérivé d'acide 3-céphem-4-carboxylique

(84) Designated Contracting States:
**AT BE CH DE DK ES FR GB GR IE IT LI LU MC NL PT SE**

(30) Priority: **05.02.1992 AT 19192**

(43) Date of publication of application: **23.08.2000 Bulletin 2000/34**

(62) Document number(s) of the earlier application(s) in accordance with Art. 76 EPC: **93901714.1 / 0 630 380**

(73) Proprietor: **Biochemie GesmbH**
**6250 Kundl (AT)**

(72) Inventors:
- **Ludescher, Johannes**
  **6252 Breitenbach (AT)**
- **Prager, Bernhard**
  **6230 Brixlegg (AT)**
- **Wolf, Siegfried**
  **6230 Brixlegg (AT)**

(74) Representative: **Grubb, Philip William et al**
**Novartis AG,**
**Corporate Intellectual Property,**
**Lichtstrasse 35**
**4002 Basel (CH)**

(56) References cited:
**EP-A- 0 503 453**
**GB-A- 2 173 798**
**US-A- 4 694 079**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 029 864 B1

## Description

The invention relates to methods of reducing the E-(trans) isomer amount in the Z/E (cis/trans) 7-amino-3-(1-propen-1-yl)-3-cephem-4-carboxylic acid of formula I

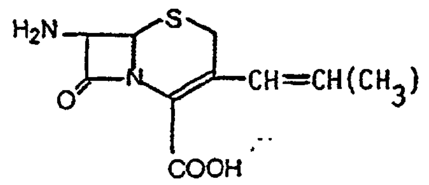

I

The Z-isomer, 7-amino-3-[(Z)-1-propen-l-yl]-3-cephem-4-carboxylic acid of formula Ia

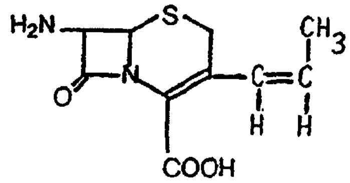

Ia

is a central intermediate for the production of highly effective broad-band antibiotics, for example cefprozil and BAY v 3522 of formulae

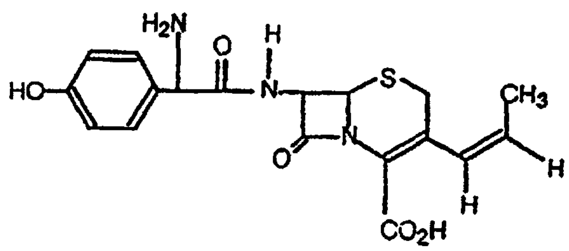

cefprozil

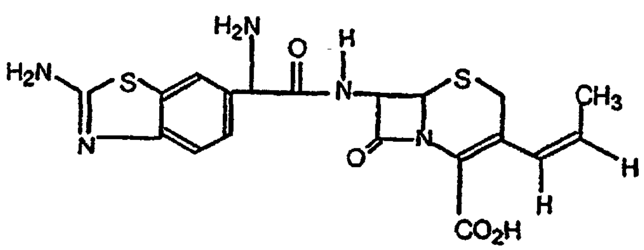

BAY v 3522

It is known that the Z-(or cis-)configuration represents the characteristic which determines the advantageous antibacterial range. Consequently, an active substance with the smallest possible proportion of E-(or trans-) isomer is desired for optimum efficiency. For example the undesired E-isomer in cefprozil should not exceed 11% according to the US Pharmocopeia.

Synthetic processes for the production of these antibiotics generally yield Z-isomers in admixture with E-isomers.

During the synthesis of the double bond from the corresponding cephalosporin nucleus by means of a Wittig reaction with acetaldehyde, the ratio of Z- to E-isomers may only be guided in the desired direction to an unsatisfactory extent : UK Patent Application 2 135 305 describes the production of cefprozil, starting with 7 -[D-2-(t-butoxycarbonylamino)-2-(p-hydroxyphenyl)acetamido]-3-cephem-4-carboxylic acid benzhydrylester. In Procedure 8 of this UK Application, the trans-isomer is separated by high performance liquid chromatography.

In USP 4 727 070, a Z/E mixture of 7-[D-2-amino-2-(Z-and E)-1-propen-1-yl)ceph-3-em-4-carboxylic acid (cefprozil) is freed from undesired E isomer by means of a reaction with acetone and subsequent re-cleavage to the active substance. Purification of a most advanced intermediate by chromatography is expensive, and purification by chemical derivation of the end product is of course the most expensive variant.

Less expensive is the production of the relatively isomer-free intermediate product, namely 7-amino-3-[(Z)-1-propen-1-yl]-3-cephem-4-carboxylic acid. However, the necessary olefinization reaction may not progress sufficiently selectively to the desired Z-compound. For example, in UK Patent Application 2 173 798 or similarly in US 4,694,079, 7-amino-3-(1-propen-1-yl)-3-cephem-4-carboxylic acid benzhydryl ester.hydrochloride is obtained for example as a maximum 90/10 Z/E mixture in a Wittig reaction as an intermediate stage to the antibiotic cefprozil. From this, ester cleavage takes place to produce 7-amino-3-[(Z)-1-propen-1-yl)-3-cephem-4-carboxylic acid with a proportion of E-isomer of 9.7%. In procedure 8 of GB-A-2173798, the ratio of Z/E was 83/17. In EP-A-292 806, 7-amino-3-(1-propen-1-yl)-3-cephem-4-carboxylic acid is produced with a maximum Z/E ratio of 91/9. Further purification at the stage of this central intermediate product is not described. Wittig reaction as an intermediate stage to the antibiotic cefprozil. From this, ester cleavage takes place to produce 7-amino-3-[(Z)-1-propen-1-yl)-3-cephem-4-carboxylic acid with a proportion of Z-isomer of 9.7%. In Example 4, the ratio of Z/E is 83/17. In EP-A-0 292 806, 7-amino-3-(1-propen-1-yl)-3-cephem-4-carboxylic acid is produced with a maximum Z/E ratio of 91/9. Further purification at the stage of this central intermediate product is not described.

It has been surprisingly found that Z/E 7-amino-3-(1-propen-1-yl)-3-cephem-4-carboxylic acid of formula I with a too high E proportion may be converted into compound of formula I with low E content via the hydrochloride of the compound of formula I.

In particular the invention comprises simple and efficient methods of depleting 7-amino-3-[(E)-1-propen-1-yl]-3-cephem-4-carboxylic acid in Z/E mixtures of 7-amino-3-[1-propen-1-yl]-3-cephem-4-carboxylic acid by

a) forming the hydrochloride of the compound of formula I'

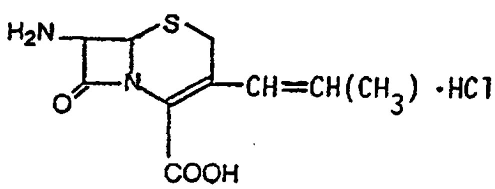

I'

wherein the propenyl group has Z- and E-configuration, from compound of formula I and hydrochloric acid in a solvent or solvent mixture, in which the Z- and E-isomers of formula I' have different solubilities
or
solubilising at least part of 7-amino-3-[(E)-1-propen-1-yl] -3-cephem-4-carboxylic acid hydrochloride in Z/E mixtures of the compound of formula I' in a solvent or solvent mixture, in which the Z- and E-isomers have different solubilities, and recovering the enriched Z-isomer of formula I',
and optionally converting the obtained compound of formula I' into the free compound of formula I with a reduced E-amount by adjusting the pH or

Process a) may be carried out as follows:

The formation of the hydrochloride is carried out in a solvent or solvent mixture, in which the Z- and E-isomers of formula I' have different solubilities.

The hydrochloride of formula I' may be formed both in protic and in aprotic medium. If operating in an aqueous system, the Z/E mixture of the compound of formula I is dissolved in water or an aqueous organic solvent such as aqueous alcohol, e.g. isopropanol, aqueous acetone or acetonitrile, by adding hydrochloric acid, and precipitating the hydrochloride by addition of an organic counter solvent. Suitable organic counter solvents are in particular organic nitriles such as acetonitrile; ketones, e.g. acetone; alcohols, e.g. methanol, ethanol, one of the isomeric propanols or butanols; ethers, e.g. tert.butyl methyl ether, diethylether or tetrahydrofuran or esters, e.g. ethyl or isopropyl acetate or mixtures thereof. If operating in a practically water-free system, the Z/E mixture of formula I is dissolved in concentrated form in an alcohol, e.g. methanol or isopropanol, with water-free hydrochloric acid, and is then diluted with one of the above mentioned counter solvents, whereby the crystalline 2-enriched product is crystallized out.

According to another variant, the hydrochloride of formula I' may be suspended or dissolved in a solvent or solvent mixture in which the E-isomer of 7-amino-3-(1-propen-1-yl)-3-cephem-4-carboxylic acid hydrochloride is better

soluble than the corresponding Z-isomer. Suitable solvents are alcohols, e.g. methanol, ethanol or isopropanol or a sulfone, e.g. sulfolane. Precipitation is then induced by e.g. adjustment of the solubility product of the Z- or E-isomer optionally by addition of one of the above mentioned counter solvent, whereby the hydrochloride of compound of formula I' with a reduced E-amount is obtained.

The hydrochloride which is thereby much improved in its Z/E ratio may subsequently be converted again into the compound of formula I in conventional manner, e.g. by means of pH adjustment in water to the approximate isoelectric point.

If a mixture of Z and E 7-amino-3-(1-propen-1-yl)-3-cephem-4-carboxylic acid of formula I is dissolved in water in an alkali or an acid and is then reprecipitated by setting the pH at the isoelectric point, there is no improvement, but rather a worsening of the Z/E ratio (see comparison tests in Examples 11a and 11b). On the other hand, using the crystalline hydrochloride of 7-amino-3-(1-propen-1-yl)-3-cephem-4-carboxylic acid of formula I', the Z/E ratio is surprisingly considerably improved. The depletion rates of undesired E-compound are up to 7%.

Compounds of formula I' may be prepared as described by the processes herein containing various amounts of Z/E isomers, e.g. in a ratio of 91:9 or less, 92:8 or less, conveniently 94:6 or more, or 95:5 or less; 97:3 or less; 99:1 or less.

The crystalline hydrochloride of formula I' is new and also form part of the invention. The hydrochloride of formula I' having a Z/E ratio of at least 91:9 or less is also new and form part of the invention.

Process a), is suitable for industrial scale.

In the following Examples, which illustrate the invention more fully, but in no way limit its scope, all temperatures are given in degrees celsius.

in the Examples the following abbreviations are used:

PACA = 7-amino-3-[(Z/E)-1-propen-1-yl]-3-cephem-4-carboxylic acid

**1. <u>Production of E-depleted 7-amino-3-[(Z/E)-1-propen-1-yl]-3-cephem-4-carboxylic acid.hydrochloride [process a]</u>**

**Example 1a)**

A mixture of 15 ml of 5 N HCl in methanol and 30 ml of acetonitrile is cooled with ice water. 15 g of PACA (Z/E ratio = 85.4/14.6) are introduced into the solution. After stirring for 5 minutes, a solution is obtained. The cooling bath is removed and the solution is slowly diluted over the course of 30 minutes with the dropwise addition of 300 ml of acetonitrile. After adding ca. 70 ml of acetonitrile, the solution is seeded with PACA.hydrochloride. The resultant crystal suspension is stirred for a further one hour whilst cooling with ice. The product is isolated through a suction filter, washed twice, each time with 40 ml of acetonitrile, and dried in a vacuum drying chamber.

| | |
|---|---|
| Yield | 14.1 g |
| Chloride | 12.8% |
| Z/E ratio | 89.9/10.1 |

**Example 1b)**

10 ml of acetonitrile and 5 ml of aqueous concentrated HCl are cooled with ice water, and 5 g of PACA with a Z/E ratio of 85.4/14.6 are introduced whilst stirring. After ca. 20 minutes, a solution is obtained. The solution is slowly mixed with 100 ml of acetonitrile, whereby after the addition of 30 ml of the acetonitrile, the solution is seeded with the hydrochloride of PACA. After completion of the acetonitrile addition, the resultant crystal suspension is stirred for a further one hour whilst cooling with ice, and subsequently filtered. The hydrochloride is subsequently washed twice, each time with 15 ml of acetonitrile, and dried.

| | |
|---|---|
| Yield | 3.4 g |
| Z/E ratio | 92.8/7.2 |

**Example 1c)**

1 g of PACA.hydrochloride with a Z/E ratio of 89.1/10.1 is treated with 2 ml of methanol, whereby a solution is briefly obtained. Crystallization rapidly re-occurs. The suspension is slowly mixed with 20 ml of acetonitrile. The crystal suspension is stirred for a further 1 hour whilst cooling with ice, and subsequently filtered. The product is washed twice, each time with 5 ml of acetonitrile, and dried.

| | |
|---|---|
| Yield | 0.6. g |
| Z/E ratio | 95.5/4.5 |

**Example 1d)**

1 g of PACA.hydrochloride with a Z/E ratio of 89.9/10.1 is suspended per 2 ml of methanol, ethanol and isopropanol. After stirring for one hour and cooling with ice, the solid substance is isolated in the usual way, and dried.

| | Z/E ratio: |
|---|---|
| a) methanol | 97.2/2.8 |
| b) ethanol | 91.1/8.9 |
| c) isopropanol | 92.5/7.5 |

**2. <u>Conversion of 7-amino-3-[(Z/E)-1-propen-1-yl]-3-cephem-4-carboxylic acid, hydrochloride into 7-amino-3-[(Z/E)-1-propen-1-yl]-3-cephem-4-carboxylic acid</u>**

**Example 2:**

2 g of PACA.hydrochloride with a Z/E ratio of 89.9/10.1 are introduced into 20 ml of water. The pH value of the suspension is 0.9. The suspension is carefully adjusted to pH 3.5 with 2 N NaOH. The suspension is stirred for a further 1 hour whilst cooling with ice, the product is filtered off, washed twice, each time with 5 ml of water, and dried.

| | |
|---|---|
| Yield | 1.7 g |
| Z/E ratio | 89.7/10.3 |

**11. <u>Comparison tests for depletion, by dissolving and reprecipitating 7-amino-3-(1-propen-1-yl)-3-cephen-4-carboxylic acid</u>**

**Example 11a)**

3 g of PACA with a Z/E ratio of 85.4/14.6 are suspended in 30 ml of water. Ca. 6 ml of concentrated HCl are added whilst cooling with ice, until a solution is obtained. The pH value is subsequently adjusted to pH 3.5 by carefully adding 5 N NaOH without external cooling. The crystal suspension is then stirred for 1 hour whilst cooling with ice, the product is filtered off, washed twice, each time with 10 ml of water, and dried in a vacuum drying chamber.

| | |
|---|---|
| Yield | 2.8 g |
| Z/E ratio | 84.5/15.5 |

**Example 11b)**

3 g of PACA with a Z/E ratio of 82.6/17.6 are suspended in 50 ml of water. 5 N NaOH (ca. 2.5 ml) is added whilst stirring until a solution is obtained (pH 8.7). The pH is subsequently adjusted to pH 3.5 by carefully adding concentrated HCl, diluted to 1/1. The crystal suspension is stirred for a further 1 hour whilst cooling with ice, and subsequently isolated and dried as described in Example 3a).

| | |
|---|---|
| Yield | 2.9 g |
| Z/E ratio | 81.8/18.2 |

## Claims

1. Process for the depletion of 7-amino-3-[(E)-1-propen-1-yl]-3-cephem-4-carboxylic acid in mixtures of 7-amino-3-[(Z)-1-propen-1-yl]-3-cephem-4-carboxylic acid and 7-amino-3-[(E)-1-propen-1-yl]-3-cephem-4-carboxylic acid which comprises forming the hydrochloride of the compound of formula I'

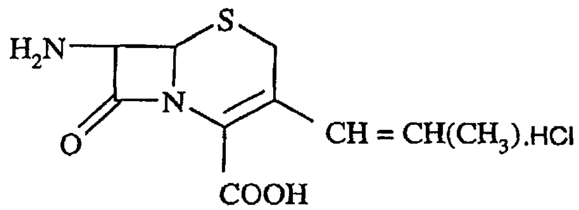

I'

wherein the propenyl group has Z- and E-configuration, from compound of formula I

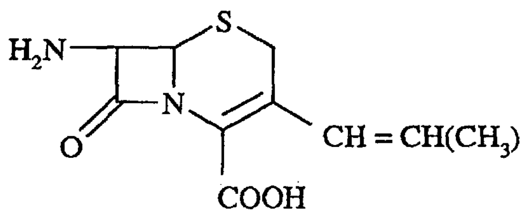

I

wherein the propenyl group has Z- and E-configuration, and hydrochloric acid in a solvent or solvent mixture, in which the Z- and E-isomers of formula I' have different solubilities, or
solubilising at least part of 7-amino-3-[(E)-1-propen-1-yl]-3-cephem-4-carboxylic acid hydrochloride in Z/E mixtures of the compound of formula I' in a solvent or solvent mixture, in which the Z-and E-isomers have different solubilities and recovering the enriched Z-isomer of formula I',
and optionally converting the obtained compound of formula I' into the free compound of formula I with a reduced E-amount by adjusting the pH.

2. A process according to claim 1 wherein the enriched Z-isomer of formula I' is recovered from the solution by adjusting the solubility product optionally by adding a counter solvent.

3. Use of a compound of formula I' in a process for the depletion of 7-amino-3-[(E)-1-propen-1-yl]-3-cephem-4-carboxylic acid in mixtures of 7-amino-3-[(Z)-1-propen-1-yl]-3-cephem-4-carboxylic acid and 7-amino-3-[(E)-1-propen-1-yl]-3-cephem-4-carboxylic acid according to claim 1.

## Patentansprüche

1. Verfahren zur Depletion von 7-Amino-3-[(E)-1-prepen-1-yl]-3-cephem-4-carbonsäure in Gemischen aus 7-Amino-3-[(Z)-1-propen-1-yl]-3-cephem-4-carbonsäure und 7-Amino-3-[(E)-1-propen-1-yl]-3-cephem-4-carbonsäure, durch Bildung des Hydrochlorids der Verbindung der Formel I'

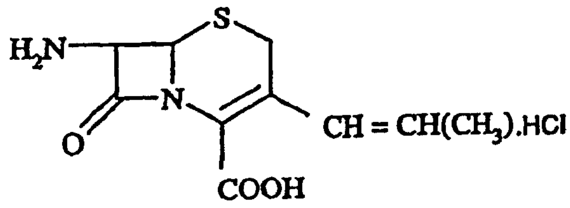

I'

worin die Propenylgruppe die Z- und E-Konfiguration hat, aus der Verbindung der Formel I

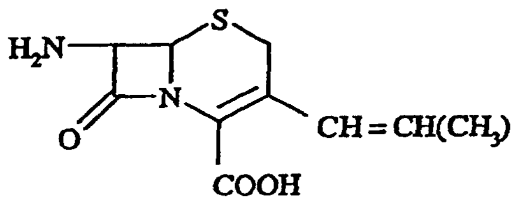

I

worin die Propenylgruppe die Z- und E-Konfiguration hat, mit Chlorwasserstoffsäure in einem Lösemittel oder Lösemittelgemisch, in welchem die Z- und E-Isomeren der Formel I' unterschiedliche Solubilitäten haben, oder durch Solubilisation wenigstens eines Teils des 7-Amino-3-[(E)-1-propen-1-yl]-3-cephem-4-carbonsäurehydrochlorids in Z/E Gemischen der Verbindung der Formel r in einem Lösemittel oder Lösemittelgemisch, in welchem die Z- und E-Isomeren unterschiedliche Solubilitäten haben, und durch Gewinnung des angereicherten Z-Isomers der Formel I' und durch optionale Umwandlung der erhaltenen Verbindung der Formel I' in die freie Verbindung der Formel I mit einer reduzierten Menge an E-Isomer durch Einstellung des pH-Wertes.

**2.** Verfahren nach Anspruch 1, worin die an Z-Isomer angereicherte Verbindung der Formel I' aus der Lösung durch Einstellung des Solubilitätsproduktes durch optionalen Zusatz eines Gegenlösemittels gewonnen wird.

**3.** Verwendung einer Verbindung der Formel r in einem Verfahren zur Depletion von 7-Amino-3-[(E)-1-propen-1-yl]-3-cephem-4-carbonsäure in Gemischen aus 7-Amino-3-[(Z)-1-propen-1-yl]-3-cephem-4-carbonsäure und 7-Amino-3-[(E)-1-propen-1-yl]-3-cephein-4-carbonsäure gemäß Anspruch 1.

**Revendications**

**1.** Procédé d'appauvrissement de l'acide 7-amino-3-[(E)-1-propén-1-yl]-3-céphème-4-carboxylique dans des mélanges d'acide 7-amino-3-[(Z)-1-propén-1-yl]-3-céphème-4-carboxylique et d'acide 7-amino-3-[(E)-1-propén-1-yl]-3-céphème-4-carboxylique, qui comprend la formation du chlorhydrate du composé de formule I':

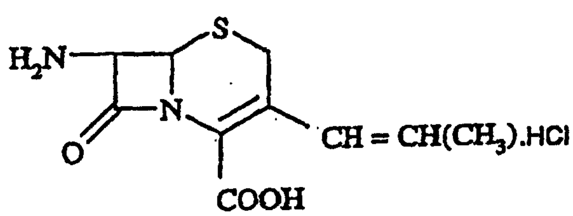

I'

dans laquelle le groupe propényle a une configuration Z et E, à partir du composé de formule I:

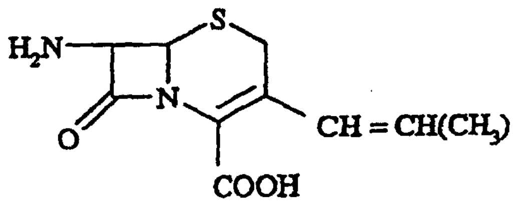

I

dans laquelle le groupe propényle a une configuration Z et E, et d'acide chlorhydrique dans un solvant ou un mélange de solvants, les isomères Z et E de formule I' ayant des solubilités différentes, ou
en solubilisant au moins une partie du chlorhydrate de l'acide 7-amino-3-[(E)-1-propén-1-yl]-3-céphème-4-carboxylique dans les mélanges Z/E du composé de formule I' dans un solvant ou un mélange de solvants, les isomères Z et E ayant des solubilités différentes, et en récupérant l'isomère Z enrichi de formule I',
et en transformant facultativement le composé obtenu de formule I' en le composé libre de formule I ayant une teneur réduite en E en ajustant le pH.

**2.** Procédé suivant la revendication 1, dans lequel l'isomère Z enrichi de formule I' est récupéré à partir de la solution en ajustant la solubilité du produit, facultativement par addition d'un contre-solvant.

**3.** Utilisation d'un composé de formule I' dans un procédé pour l'appauvrissement de l'acide 7-amino-3-[(E)-1-propén-1-yl]-3-céphème-4-carboxylique dans des mélanges d'acide 7-amino-3-[(Z)-1-propén-1-yl]-3-céphème-4-carboxylique et d'acide 7-amino-3-[(E)-1-propén-1-yl]-3-céphème-4-carboxylique conformément à la revendication 1.